# EUROPÄISCHE PATENTSCHRIFT

(11) **EP 2 917 273 B1**
(45) Veröffentlichungstag und Bekanntmachung des Hinweises auf die Patenterteilung: **28.08.2019**
(21) Anmeldenummer: 13802258.7
(22) Anmeldetag: 08.11.2013
(51) Int. Cl.: C08J 11/10

(54) **VERFAHREN UND VORRICHTUNG ZUR BEHANDLUNG VON POLYMEREN**
METHOD AND DEVICE FOR TREATING POLYMERS
PROCÉDÉ ET DISPOSITIF DE TRAITEMENT DE POLYMÈRES

(30) Priorität: 09.11.2012 DE 102012220498
(43) Veröffentlichungstag der Anmeldung: 16.09.2015
(73) Patentinhaber: Aquafil Engineering GmbH, 13469 Berlin (DE)
(72) Erfinder: KARASIAK, Wolf, 13467 Berlin (DE); KARASIAK, Dirk, 16548 Glienicke/Nordbahn (DE)
(74) Vertreter: Maikowski & Ninnemann Patentanwälte Partnerschaft mbB
(86) Internationale Anmeldenummer: PCT/EP2013/073430
(87) Internationale Veröffentlichungsnummer: WO 2014/072483

(56) Entgegenhaltungen:
- EP-A1- 0 522 235
- EP-A1- 1 631 538
- WO-A1-00/47659
- WO-A1-2007/063462
- US-A- 5 432 203
- US-A1- 2005 096 482

## Beschreibung

Die vorliegende Erfindung betrifft ein kontinuierliches Verfahren zur Depolymerisation eines inhomogenen Polymergemisches gemäß Anspruch 1 1 und eine Vorrichtung zur Durchführung solch eines Verfahrens gemäß Anspruch 11.

### Beschreibung

Polymerabfälle, welche beim Herstellungsprozess von Fäden, Fasern, Filmen, Flaschen etc. oder bei der Polymerherstellung selbst anfallen, werden schon seit Langem in der Industrie verarbeitet. Hierbei wird jedoch darauf geachtet, dass der Abfall möglichst rein gehalten und nicht verunreinigt wird. Der Anteil dieses sauberen Abfalls am Gesamtaufkommen des Abfalls ist jedoch relativ gering.

Der größere Anteil ist der sogenannte post-consumer Abfall, also der Abfall, der nach Verwendung beim Verbraucher anfällt, erheblich verschmutzt sein kann und bisher in großen Mengen auf Deponien oder in Landauffüllungen abgelagert wird. Durch diese Vorgehensweise werden große Rohstoffmengen und Ressourcen nicht genutzt.

In den bisher bekannten Verfahren zur Aufarbeitung von Polymerabfällen erfolgt z.B. die Rückgewinnung von Polyester, hergestellt aus einer Dicarbonsäure und einem Diol, durch diskontinuierliche oder halbkontinuierliche Verfahren. Hierzu müssen Fremdstoffe wie andere Polymere, Papier, Klebstoffe, Schmutz etc. aus Abfallstoff entfernt werden.

Die Entfernung von Farbstoffen aus Polymerabfällen erfolgt bisher gar nicht oder nur äußerst begrenzt. Aus diesem Grund wird der Abfall vorsortiert und farbliche Abfälle werden einer gesonderten Verwendung zugeführt bzw. werden erneut in den Abfall gegeben.

Ein weiterer Teil der nicht farbigen post-consumer Abfälle wird in Waschanlagen von Fremdstoffen befreit und in meist mechanischen Anlagen, z.B. Extrudern, einer neuen, meist down-recycelten Verwendung zugeführt.

Bei Abfällen aus Polyamid (Nylon 6), werden die Moleküle in einem diskontinuierlichen Prozess unter hohem Druck und Verwendung von überhitztem Wasserdampf und Säure aufgespalten. Anschließend wird das erhaltene verunreinigte Caprolactam einer aufwendigen Reinigung unterzogen, bis es erneut für die Herstellung von Polyamid eingesetzt werden kann.

Aufgrund der diskontinuierlichen bzw. halbkontinuierlichen Recycling-Verfahren sind deren Leistungen beschränkt und für den Einsatz bei großen Abfallmengen, welche z.B. im Bereich von Polyester-Flaschen anfallen, unwirtschaftlich.

Bei Polyesterabfällen wird das Abfall-Polymer typischerweise in Batch-Verfahren z.B. zu dem Monomer Bis(hydroxyethyl)terephthalat (BHET) und weiteren Monomeren und Oligomeren abgebaut bzw. depolymerisiert. Ein Nachteil bei den bisher verwendeten Batch-Verfahren ist die relativ lange Verweildauer der Monomer-Lösung unter den Batch-Bedingungen, die zu einer unerwünschten Verfärbung der Monomer-Lösung und zur Erhöhung des DEG Gehaltes (Diethylenglykol) führt. Die lange Verweildauer in einem Batch-Reaktor ist in erster Linie durch die unterschiedlichen Polymerdicke und / oder -größe bedingt; je größer die Polymerpartikel desto längere Löse- bzw. Depolymerisationszeit ist notwendig. Im Umkehrschluss bedeutet dies, dass bereits vorhandene Monomere in einem Batch-Reaktor den hohen Depolymerisationstemperaturen ausgesetzt sind, und es dabei zur Bildung von unerwünschten Nebenprodukten z.B. Verfärbungen und DEG kommt, die aufwendig entfernt werden müssen oder unerwünscht sind. Dies ist mit hohen Kosten verbunden.

Außerdem führt das bisherige diskontinuierliche oder halbkontinuierliche Recycling zu starken Qualitätsschwankungen und erfordert große Lagerkapazitäten für die rückgewonnenen Stoffe. Deshalb gibt es auf diesem Gebiet auch Erfindungen zur Reinigung des hergestellten Monomers (EP1510514). Grundsätzlich wurden jedoch eher prozentuale Begrenzungen für die Verwendung des recycelten Oligomers/Monomers bei 30% bis zu 50% eingeführt, um Qualitätsschwankungen im Polymerisations-/Polykondensationsprozesse durch Zugabe von Recyclingmaterial zu vermeiden.

Die WO 00/47659 A1 offenbart ein kontinuierliches Verfahren zur Depolymerisation von PET umfassend die Schritte Einführen eines Polymergemisches mit inhomogener Partikelgrößenverteilung in einen vertikal angeordneten Reaktor mit einem Reaktoreinlass und einem Reaktorauslass, Hinzufügen von Ethylenglykol als Reaktanten und kontinuierliche Depolymerisation des inhomogenen Polymergemisches zum flüssigen Oligomeren.

Aufgrund der bisher nur unzureichenden Behandlung und Aufarbeitung von Polymeren, insbesondere von Polymerabfällen bestand daher die Aufgabe, Verfahren und Vorrichtungen zu entwickeln, die die Nachteile der bekannten Verfahren vermeiden und zugleich kosteneffektiv durchführbar sind.

Diese Aufgabe wird mit einem kontinuierlichen Verfahren gemäß dem Anspruch 1 und einer Vorrichtung gemäß dem Anspruch 11 gelöst.

Demnach wird ein kontinuierliches Verfahren zur Behandlung von inhomogenen Polymergemischen beansprucht. Die Inhomogenität bezieht sich auf die Partikelgröße und / oder Partikeldicke. Die Behandlung umfasst eine Depolymerisation eines Polymergemisches unter Ausbildung einer im Wesentlichen aus einem Monomer bestehenden Monomerflüssigkeit.

Die Monomerflüssigkeit beinhaltet u.a. mindestens ein Monomer, mindestens ein Lösungsmittel und / oder mindestens einen Reaktanten. Die Monomerflüssigkeit kann auch Oligomere und / oder Polymere enthalten.

Das Verfahren umfasst die in Anspruch 1 beschriebenen Schritte.

Das vorliegende Verfahren bewirkt somit die Ausbildung eines Gradienten entlang des Reaktors, der durch Partikeldicke und / oder Partikelgröße sowie Kettenlänge des Polymergemisches bestimmt wird. Kleine und dünne Polymerpartikel werden unmittelbar nach Eintritt in den Depolymerisationsreaktor depolymerisiert und die gebildete Monomerflüssigkeit wird ohne längere und unnötige Verweildauer aus dem Reaktor abgeführt.

Große und dicke Partikel hingegen benötigen eine längere Depolymerisationszeit bzw. Lösezeit und müssen somit länger im Reaktor bis zu einer vollständigen Depolymerisation oder Lösung verbleiben. Die großen und dicken Partikel werden daher in dem Bereich des Reaktors, der dem Reaktoreingang bzw. Reaktoreinlass am nächsten liegt, z.B. durch geeignete Mittel solange zurückgehalten, bis diese in ausreichendem Maß flüssig sind. Die dabei gebildete Monomerflüssigkeit wird hin zum Reaktorauslass geführt und am Reaktorauslass aus dem Reaktor abgeführt.

Erfindungsgemäß werden Partikel mit geringer Partikelgröße und / oder geringer Partikeldicke des inhomogenen Polymergemisches nach Eintritt in den Reaktor sofort depolymerisiert und / oder gelöst und die gebildete Monomerflüssigkeit hin zum Reaktorauslass geführt und am Reaktorauslass aus dem Reaktor abgeführt, und wobei Partikel großer Partikelgröße und / oder Partikeldicke des inhomogenen Polymergemisches nach Eintritt in den Reaktor solange im Reaktor verweilen, bis diese Partikel des Polymergemisches vollständig depolymerisiert und / oder gelöst sind.

Erfindungsgemäß werden innerhalb des Reaktors mindestens zwei Reaktionszonen herausgebildet. Diese Reaktionszonen werden durch geeignete Einbauten gebildet durch welche das gröbere Polymermaterial (große und dicke oder nicht lösliche oder nicht polymerisierbare Partikel) zurückgehalten wird, aber durch welche gleichzeitig die gebildete Monomerflüssigkeit aus den Reaktionszonen abfließen kann. Vorstellbar wären z.B. mit Löchern versehene Platten oder ähnliches. Es kann dabei auch vorgesehen sein, dass derartige Löcher in den Platten am Reaktoreinlass einen größeren Durchmesser aufweisen als am Reaktorende. Am Reaktoreinlass könnten demnach sehr große und dicke Partikel zurückgehalten, während in Richtung Reaktorauslass kleinere und dünnere Partikel zurückgehalten werden. Da sich die Größe und Dicke der Partikel sich im Verlaufe des Prozesses ändert, insbesondere abnimmt, wandern die noch nicht vollständig verflüssigten, depolymerisierten Partikel mit abnehmender Größe in Richtung Reaktorauslass; mit fortschreitendem Prozess gelangen die kleiner und dünner werdenden Polymerpartikel von einer Reaktionszone in die nächste Reaktionszone.

Wie erwähnt, wird das vorliegende Verfahren in einem vertikalen oder horizontalen Reaktor durchgeführt.

Im Falle eines vertikalen Reaktors kann in einer Ausführungsform vorgesehen sein, dass das inhomogene Polymergemisch in diesen mindestens einen vertikalen Reaktor, der über mindestens zwei Reaktionszonen mit jeweils mindestens einer Mischeinrichtung verfügt, eingeführt wird, wobei das inhomogene Polymergemisch den Reaktor von oben nach unten durchströmt, und wobei das Polymer entsprechend gelöst oder depolymerisiert wird. Die Verwendung eines vertikalen Reaktors mit mindestens zwei Reaktionszonen, der von dem Polymer von oben nach unten durchströmt wird, ermöglicht ein Anlösen des Polymers bereits im oberen Reaktorbereich.

Im Falle eines vertikalen Reaktors kann in einer weiteren Ausführungsform auch vorgesehen sein, dass das inhomogene Polymergemisch in diesen mindestens einen vertikalen Reaktor eingeführt wird, wobei das inhomogene Polymergemisch den Reaktor von unten nach oben durchströmt, und wobei das Polymer entsprechend angelöst und depolymerisiert wird.

In dieser zweiten Verfahrensvariante im Falle eines vertikalen Reaktors kann das Polymergemisch in verschiedener Weise von unten in den Reaktor eingetragen werden. So kann das Polymergemisch am unteren Ende des Reaktors von außen zugeführt werden. Es ist aber auch denkbar, das Polymergemisch über eine Leitung in den Reaktorinnenraum einzuführen, so dass das Polymergemisch im unteren Bereich des Reaktorinnenraums aus der Leitung in denselbigen austritt. Im Falle dieser Ausführungsvariante, in welcher das Polymergemisch den Reaktor von unten nach oben durchströmt, erfolgt die Gradientenbildung entlang des Reaktors von unten nach oben. Hierbei ist nicht zwingend eine Ausbildung von Reaktionszonen erforderlich, vielmehr verweilen die größeren und dickeren Partikel des Polymergemisches aufgrund ihres größeren Gewichtes bzw. Masse im unteren Bereich des Reaktors und die Monomerflüssigkeit steigt im Reaktor nach oben (oder wird nach oben gefördert oder bewegt sich nach oben).

Wird ein horizontaler Reaktor verwendet, so weist dieser horizontale Reaktor vorzugsweise eine Misch- und / oder Fördereinrichtung, z.B. eine Förderschnecke auf, die entlang der Längsachse des Reaktors in dessen Innenraum vorgesehen ist. In diesem Falle wird das inhomogene Polymergemisch in diesen mindestens einen horizontalen Reaktor so eingeführt, dass das inhomogene Polymergemisch in den horizontalen Reaktor z.B. entgegen die gegen die Strömungsrichtung arbeitende Förderschnecke eingeleitet wird und die Monomerflüssigkeit am Reaktorende abgeführt wird. Solch ein horizontaler Reaktor weist mehrere Reaktionszonen, jedoch mindestens zwei Reaktionszonen, auf, die z.B. durch die Misch- und / oder Förderschnecke ausgebildet werden. Die Gewindegänge der Misch- und / oder Förderschnecke sind mit Löchern versehen. Die Löcher sind am Reaktoreinlass größer ausgeführt als am Reaktorauslass. Hierdurch werden während der Drehung der Schnecke die größeren und dickeren Partikel, d.h. noch nicht depolymerisierte oder nicht gelöste Partikel, stetig in Richtung zum Reaktoreinlass befördert bzw. im Bereich des Reaktoreinlasses gehalten, während die durch die Depolymerisation oder Lösung gebildete Monomerflüssigkeit durch die immer kleiner werdenden Löcher in den Gewindegängen zum Reaktorauslass gelangt. Ein Beispiel für solch einen Reaktor ist in der DE 1518908 beschrieben.

Das zu behandelnde inhomogene Polymergemisch (z.B. aus Polyestern oder Polyamiden) ist insbesondere ein Abfall-Polymergemisch, und bevorzugt ein farbiges Polymergemisch aus Industrie- und / oder Verbraucherabfällen. Typische Polyester sind z.B. Polybutylenterephthalat (PBT), Polyethylenterephthalat (PET), Polytrimethylenterephthalat (PTT), Polyethylennaphthalat (PEN) oder auch Polycarbonate (PC). Mit dem vorliegenden Verfahren wird bevorzugt PET abgebaut. Als typische Polyamide sei beispielhaft auf die Polyamide 6, 6.6 oder 6.10 hingewiesen.

In dem vorliegenden Verfahren wird das Polymer z.B. ein Abfall-Polymer somit bevorzugt kontinuierlich in einen ein- oder mehrstufigen Reaktor eingebracht, wobei das Polymer bevorzugt möglichst sortenrein ist. Häufig beschriebene Probleme mit PVC können ohne Probleme in dem Verfahren und Reaktor abgetrennt werden.

Der bisher beschriebene negative Effekt der Verfärbung bei zu hohen Temperaturen und langen Verweilzeiten im Depolymerisationsreaktor bei Verwendung von reinem ungefärbtem Polymerabfall entfällt, da das eingebrachte Polymer im Vergleich zu den bisherigen Verfahren nicht nach Farben sortiert werden muss. Es können alle Polymerfarben in dem neuen Verfahren verwertet werden.

In einer Ausführungsform wird das Polymer vor Einführen in den Reaktor zu einem inhomogenen Polymergemisch zerkleinert. Das inhomogene Polymergemisch kann Polymerpartikel mit einer Größe, insbesondere einer mittleren Größe in einem Bereich zwischen 30 mm und 1 mm, bevorzugt zwischen 15 mm und 3 mm aufweisen. Alternativ kann die mittlere Partikelgröße z.B. aus Folienabfällen zwischen 5 µm und 1 mm, insbesondere zwischen 10 µm und 0,5 mm betragen.

Nach Zerkleinerung kann das inhomogene Polymergemisch vor Zufuhr in den Reaktor mit mindestens einem Reaktant oder mit mindestens einem Lösungsmittel in Kontakt gebracht oder gemischt werden. Als Reaktant und / oder Lösungsmittel können ein organisches Lösungsmittel, insbesondere Glykole mit der allgemeinen Formel R(OH)₂ mit R = CₙH₂ₙ mit n = 2 bis 12, bevorzugt Ethylenglykol, oder eine wässrige Lösung, insbesondere eine saure wässrige Lösung verwendet werden.

Der Reaktant oder das Lösungsmittel (Flüssigkeit) zum Depolymerisieren oder Lösen des inhomogenen Polymergemisches kann sowohl im Unterschuss als auch im Überschuss zugegeben werden. Es wird jedoch bevorzugt im Unterschuss zugegeben, um die Rückgewinnung oder Reinigung der Flüssigkeit einfach sowie kostengünstig zu gestalten und um die Monomerflüssigkeit nicht zu sehr zu verdünnen.

Das inhomogene Polymergemisch wird in dem Reaktor kontinuierlich im Wesentlichen zum Monomer abgebaut oder depolymerisiert. Vorwiegend bedeutet im Wesentlichen, dass die Monomerflüssigkeit überwiegend aus einem Monomer besteht. Darüber hinaus können Oligomere mit einer Kettenlänge von weniger als 50 in der Monomerflüssigkeit nach dem Depolymerisationsprozess enthalten sein. Im Falle des Polyesters PET enthält die Monomerflüssigkeit über 90 Gew% des Monomers Bis(hydroxyethyl)terephthalat (BHET).

Dabei ist es unerheblich, ob das Polymer vorher einem Anlösevorgang unterzogen worden ist oder nicht. Der Anlösevorgang wirkt sich zwar positiv auf die Depolymerisationzeit aus, kann aber grundsätzlich durch den neuartigen kontinuierlich betriebenen Depolymerisationsreaktor übernommen werden. Zusätzlich ist eine Vorlage von bereits depolymerisierten Produkt, Monomer oder BHET, wie in diskontinuierlichen Verfahren häufig erwähnt, nicht erforderlich. In den unterschiedlichen Zonen des Depolymerisationsreaktors können die Prozessbedingungen entsprechend der unterschiedlichen Qualität und Viskosität des Rohabfalles angepasst werden.

Erfindungsgemäß weist der Reaktor mindestens zwei Reaktionszonen auf, wobei im Falle eines vertikalen Reaktors in jeder der Reaktionszonen eine Mischvorrichtung angeordnet ist. Es ist aber denkbar, dass eine oder mehrere der Reaktionszonen keine Mischvorrichtung aufweisen. Die Mischvorrichtung kann z.B. ein Rührwerk oder eine Schnecke sein. Es ist aber auch denkbar, dass die Mischvorrichtung in Form einer Mischdüse ausgebildet ist, wobei die Durchmischung dann so erfolgt, dass zumindest ein Teil der den Reaktor verlassenen Monomerlösung in die einzelnen Reaktionszonen in Form von Teilströmen rückgeführt wird. Jeder der einzelnen Reaktionszonen kann bevorzugt separat angesteuert werden.

Die Depolymerisation des inhomogenen Polymergemische wird abhängig von der Polymerart in dem Reaktor bevorzugt bei Temperaturen zwischen 50 °C bis 350 °C, insbesondere zwischen 180°C und 240 °C, und Drücken zwischen 0,05 MPa und 1 MPa (0,5 bar und 10 bar) absolut, insbesondere zwischen 0,08 MPa und 0,6 MPa (0,8 bar und 6 bar) absolut, 0,5 MPa (5 bar) absolut durchgeführt. Die

Erwärmung erfolgt dabei über eine im Reaktor und / oder außerhalb des Reaktors angeordnete Heizung.

Die durchschnittliche Verweilzeit des Polymergemisches, insbesondere der gebildeten Monomerflüssigkeit, im Reaktor beträgt 30 bis 120 min. Die Verweilzeit ist insbesondere abhängig von dem verwendeten Polymermaterial und der Partikelgröße, wie oben ausgeführt.

Die Monomerflüssigkeit kann einer Vorrichtung zum Abtrennen von unlöslichen Teilen bzw. Stoffen, beispielsweise einer Filtervorrichtung, zugeführt werden. Die hierfür verwendeten Einrichtungen sind dem Fachmann geläufig. Diese Vorrichtung oder Einrichtung kann im Seitenstrom des Reaktors angeordnet sein.

In einer weiteren Variante des Verfahrens wird die den Reaktor verlassene Monomerflüssigkeit in mindestens einen Entfärbungsreaktor zur Entfärbung der Monomerflüssigkeit eingeführt. Es ist denkbar, dass die den Reaktor verlassene Monomerflüssigkeit zunächst von unlöslichen Stoffen befreit wird, bevor diese Monomerflüssigkeit in den Entfärbungsreaktor eintritt.

In einer Variante des Verfahrens erfolgt die Entfärbung durch ein Additiv oder Entfärbungsmittel in einem Entfärbungsreaktor. Die zur Entfärbung verwendeten Additive/Entfärbungsmittel sind ausgewählt aus einer Gruppe, enthaltend aktivierten Kohlenstoff, aktivierte Tonmineralien, wie beispielsweise Clay, Bentonit, Montmorillonit, Zeolithe. Die Additive/Entfärbungsmittel können je nach dem zu recycelnden Polymertyp in Zusammensetzung und Art variieren. Die Additive und / oder Entfärbungsmittel liegen bevorzugt als stationäre Phase wie z.B. in Form einer Membran in dem Entfärbungsreaktor vor. Ein entscheidender Vorteil des neuen Verfahrens ist es, dass eine aufwendige Abtrennung der aktiven Entfärbungsmittel durch Filtration, wie in anderen Verfahren beschrieben, nicht erforderlich ist.

In einer weiteren Variante des Verfahrens wird der Reaktant oder das Lösungsmittel von der Monomerflüssigkeit in einer Trennvorrichtung, insbesondere in einer Vorrichtung zum Eindicken und / oder Konzentrieren, abgetrennt. Hierbei kann der Reaktant oder das Lösungsmittel z.B. mittels Verdampfen in einer Verdampfungsvorrichtung von der Monomerflüssigkeit abgetrennt werden. Die Verdampfung bzw. das Abtrennen der Monomerflüssigkeit erfolgt hier bei Drücken und Temperaturen, die auf den verwendeten Reaktanten oder das Lösungsmittel abgestimmt sind.

Die in der Trennvorrichtung aufkonzentrierte Monomerflüssigkeit kann anschließend in mindestens eine Polymerisationsvorrichtung zur Herstellung eines auf den Monomeren basierenden Polymers eingespeist werden. Das vorliegende Verfahren ermöglicht demnach eine kontinuierliche Bereitstellung eines Monomers für eine anschließende erneute Polymerisationsreaktion.

Es ist auch denkbar, dass die Monomerflüssigkeit in nicht aufkonzentrierter Form in mindestens eine Polymerisationsvorrichtung zur Herstellung eines auf den Monomeren basierenden Polymers eingeführt wird. Entsprechend kann also die Monomerflüssigkeit auch unmittelbar zur Polymerisation verwendet werden ohne die Notwendigkeit weiterer kostenintensiver Aufarbeitungsschritte.

Es ist generell auch möglich, dass der den Entfärbungsreaktor verlassende Strom aus Monomerflüssigkeit aufgeteilt wird, wobei ein erster Teil unmittelbar in die Polymerisationsvorrichtung eingespeist wird und der andere, zweite Teil zunächst der Trennvorrichtung zugeführt und aufkonzentriert wird. Die aufkonzentrierte Monomerflüssigkeit kann dann anschließend ebenfalls der Polymerisationsvorrichtung zugeführt werden.

In einer weiteren Ausgestaltung des vorliegenden Verfahrens ist vorgesehen, die nichtaufkonzentrierte und / oder aufkonzentrierte Monomerflüssigkeit in mindestens einer weiteren Einrichtung oder Vorrichtung zu verfestigen. Die Verfestigung der Monomerflüssigkeit kann z.B. auf einer Kühlwalze oder auf einem Kühlband zu Schuppen oder anders geformten Teilchen erfolgen. Das verfestigte Produkt kann abschließend abgepackt und gelagert werden und später zur Polymer-Herstellung verwendet werden.

Es ist bevorzugt, wenn das mindestens eine in der Trennvorrichtung abgetrennte Reaktant oder Lösungsmittel zur Herstellung einer Mischung mit dem insbesondere zerkleinerten inhomogenen Polymergemisch recycelt bzw. rückgeführt wird. Somit ermöglicht das vorliegende Verfahren eine kontinuierliche und ökologische Fahrweise, da der Flüssigkeitsträger wiederverwendet wird.

Es ist weiterhin bevorzugt, wenn das in der Verdampfungsvorrichtung abgetrennte Reaktant oder Lösungsmittel vor Rückführung in mindestens einer Reaktant- oder LösungsmittelAufbereitung z.B. mittels Destillation behandelt wird.

Das vorliegende kontinuierliche Verfahren kann in einer Vorrichtung mit den Merkmalen des Anspruchs 11 durchgeführt werden, die mindestens einen vertikalen oder horizontalen Reaktor zur Depolymerisation oder Lösung eines inhomogenen Polymergemisches umfasst und mindestens eine Reaktionszone aufweist.

Vorteilhafterweise weist der Reaktor mindestens eine Misch- und / oder eine Fördereinrichtung aufweist.

Erfindungsgemäß weist eine Reaktionszone eine Schnecke auf oder wird durch eine Schnecke gebildet. So kann der vertikale Reaktor mindestens zwei Reaktionszonen aufweisen, wobei in jeder Reaktionszone mindestens ein Rührwerk vorgesehen ist. Das Rührwerk ist eine Schnecke. Für den Fall, dass die Strömung der Momomerflüssigkeit von unten nach oben erfolgt, kann eine Reaktionszone ausreichen. Ein Rührwerk ist dann u.U. nicht erforderlich.

Dabei ist die Schnecke mit Löchern versehen, wobei die Größe der Löcher in Richtung des Einlassendes des Reaktors zunimmt. Beim Einlass werden die Polymerteile z.B. aus Kunststoffabfall noch relativ groß sein, so dass diese ab einer bestimmen Entfernung vom Einlass durch hinreichend kleine Löcher aufgehalten werden, damit eine effiziente Depolymerisation erfolgen.

Auch ist es vorteilhaft, wenn der Reaktor horizontal angeordnet ist, einen runden Querschnitt hat und die Schnecke exzentrisch zum Reaktor liegt. Die exzentrische Ausführung erlaubt die Bildung eines Gasraumes, was das Abziehen von entstehenden Dämpfen ermöglicht.

Die Vorrichtung kann vorteilhafterweise eine Einrichtung zum Abtrennen von unlöslichen Substanzen aufweisen. Die Abtrennung von unlöslichen Substanzen kann im Reaktor und / oder durch eine Filtereinrichtung nach dem Reaktor erfolgen.

Auch kann mindestens ein nachgeschalteter Entfärbungsreaktor zum Entfärben der Monomerflüssigkeit vorgesehen sein.

Ferner kann die Vorrichtung vorteilhafterweise eine nachgeschaltete Verfestigungsvorrichtung für die entfärbte Monomerflüssigkeit aufweisen. Die Vorrichtung zum Verfestigen des Monomers kann eine Kühlwalze oder ein Kühlband umfassen.

In einer weiteren Ausführungsform ist mindestens eine Trennvorrichtung, insbesondere zum Eindicken und / oder Konzentrieren, zum zumindest teilweisen Entfernen des Reaktanten oder Lösungsmittel aus der Monomerflüssigkeit vorgesehen. Hier wird insbesondere der Reaktant oder das Lösungsmittel aus der den Entfärbungsreaktor verlassenen farbstofffreien Monomerflüssigkeit abgetrennt und die Monomerflüssigkeit aufkonzentriert.

Auch ist es vorteilhaft, wenn eine Vorrichtung zur Aufbereitung des zumindest teilweise in der Trennvorrichtung entfernten Reaktanten oder Lösungsmittels vorgesehen ist.

In einer weiteren Variante weist die vorliegende Vorrichtung mindestens eine Vorrichtung zur Aufbereitung des zumindest teilweise in der Verdampfungsvorrichtung und / oder dem Reaktor, insbesondere dem Dempolymerisationsreaktor entfernten Reaktanten oder Lösungsmittels auf.

Der aufbereitete Flüssigkeitsträger (Reaktant oder Lösungsmittel) wird anschließend direkt oder indirekt von der Reaktant- oder Lösungsmittelaufbereitungsvorrichtung in die Zuführvorrichtung für den Reaktor rückgeführt. Es ist auch denkbar, dass der Flüssigkeitsträger (Reaktant oder Lösungsmittel) ohne Aufbereitung von der Trennvorrichtung unmittelbar zurückgeführt wird. Auf jeden Fall ist eine vollständiges Recyclen des verwendeten Reaktanten oder Lösungsmittels möglich.

Prinzipiell sind sämtliche für die Verfahren dargestellten Ausführungsvarianten auf die vorliegenden Verfahren und Vorrichtungen übertragbar und miteinander kombinierbar.

Die Erfindung wird nachfolgend unter Bezugnahme auf die Figuren der Zeichnungen an mehreren Ausführungsbeispielen näher erläutert. Es zeigen:
- Fig. 1: eine schematische Darstellung einer ersten Ausführungsform eines nicht erfindungsgemäßen Verfahrens;
- Fig. 2: eine schematische Darstellung einer zweiten Ausführungsform eines nicht erfindungsgemäßen Verfahrens; und
- Fig. 3: eine schematische Darstellung einer dritten Ausführungsform eines nicht erfindungsgemäßen Verfahrens;
- Fig. 4: eine schematische Darstellung einer vierten Ausführungsform eines nicht erfindungsgemäßen Verfahrens.

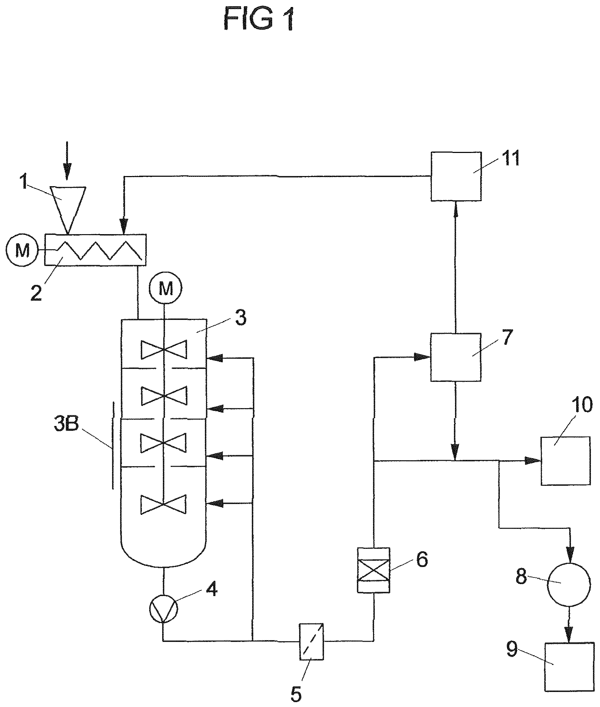

Figur 1 zeigt eine Vorrichtung bzw. Anlage zur Durchführung einer ersten Variante eines nicht erfindungsgemäßen Verfahrens.

Hierzu wird ein Polymer, bevorzugt ein Abfall-Polymer, zunächst in ein inhomogenes Polymergemisch mit einer Partikelgröße kleiner als 30 mm in einer entsprechenden Mahlvorrichtung (nicht gezeigt) zerkleinert und über eine Aufgabevorrichtung 1 in eine Zuführeinrichtung 2 eingespeist, in welcher das inhomogene Polymergemisch mit einem geeigneten Reaktant oder Lösungsmittel wie z.B. Ethylenglykol vermischt wird.

Die Mischung aus Polymergemisch und Reaktant oder Lösungsmittel wird anschließend in einen vertikalen Reaktor, insbesondere einem Depolymerisationsreaktor 3 mit mehreren Reaktionszonen über die Zuführeinrichtung 2 eingespeist. Vorliegend weist der Depolymerisationsreaktor 3 ein Rührwerk auf, das sich entlang der gesamten Reaktorlänge erstreckt. Die Mischung strömt hier von oben nach unten durch den Depolymerisationsreaktor 3.

Im Depolymerisationsreaktor 3 erfolgt der Abbau des Abfall-Polymers, z.B. Polyester zu BHET Monomer, bei einer Temperatur in einem Bereich zwischen 50°C und 350°C und einem Druck in einem Bereich zwischen 0,05 MPa und 0,5 MPa (0,5 bar und 5 bar). Die Erwärmung erfolgt dabei über eine innen- und außenliegende geeignete Heizung, beispielsweise 3B. Das im Reaktor gebildete Monomer, bestehend aus reinem BHET, Oligomeren und Reaktant wird am unteren Ende des vertikalen Reaktors 3 abgeführt.

Die den Reaktor 3 verlassene Monomerflüssigkeit wird anschließend in einen ersten Strom und einen zweiten Strom aufgespaltet, wobei der erste Strom in Form von Teilströmen in die Reaktionszonen des Reaktors 3 rückgeführt wird und der zweite Strom einer Einrichtung 5 zur Entfernung von Feststoffen zugeführt wird.

Nach Entfernung der Feststoffe wird die Monomerflüssigkeit in einen Entfärbungsreaktor 6 eingeleitet, in welchem durch aktivierte Additive/Entfärbungsmittel wie Kohlenstoff oder Ton (clay) die Farbstoffe aus der Monomerflüssigkeit entfernt werden.

Die den Entfärbungsreaktor 6 verlassene nunmehr farbstofffreie Monomerflüssigkeit wird wiederum in zwei Teilströme aufgeteilt, wobei ein erster Teilstrom der farbstofffreien Monomerflüssigkeit unmittelbar in einen Polymerisationsreaktor 10 zur Polymerherstellung eingeleitet wird und ein weiterer Teilstrom in eine Verdampfungs- bzw. Aufkonzentrierungsvorrichtung 7 zur Abtrennung des Reaktanten oder Lösungsmittels eingeleitet wird.

Die in der Verdampfungsvorrichtung 7 aufkonzentrierte farbstofffreie Monomerflüssigkeit wird anschließend entweder in den Polymerisationsreaktor 10 zur Polymerherstellung eingespeist und / oder in eine Kühlvorrichtung 8 wie z.B. eine Kühlwalze oder Kühlband zur Verfestigung eingeführt. Das verfestigte Produkt kann anschließend zweckmäßig in einer Verpackungsvorrichtung 9 verpackt werden.

Der in der Verdampfungsvorrichtung 7 entfernte Reaktant oder das entfernte Lösungsmittel wird in einer Aufbereitungsvorrichtung 11 weiteren Reinigungsschritten z.B. mittels Destillation oder ähnlichem unterzogen und anschließend unmittelbar oder nach Zwischenlagerung der Zuführeinrichtung 2 wiederzugeführt und hier mit dem Polymer-Abfall gemischt.

Das vorliegende Verfahren ermöglicht somit einen kontinuierlichen und effizienten Betrieb zur Aufarbeitung von Polymerabfällen, ohne dass eine aufwändige Trennung der Polymerabfälle entsprechend ihres Farbgehaltes notwendig ist.

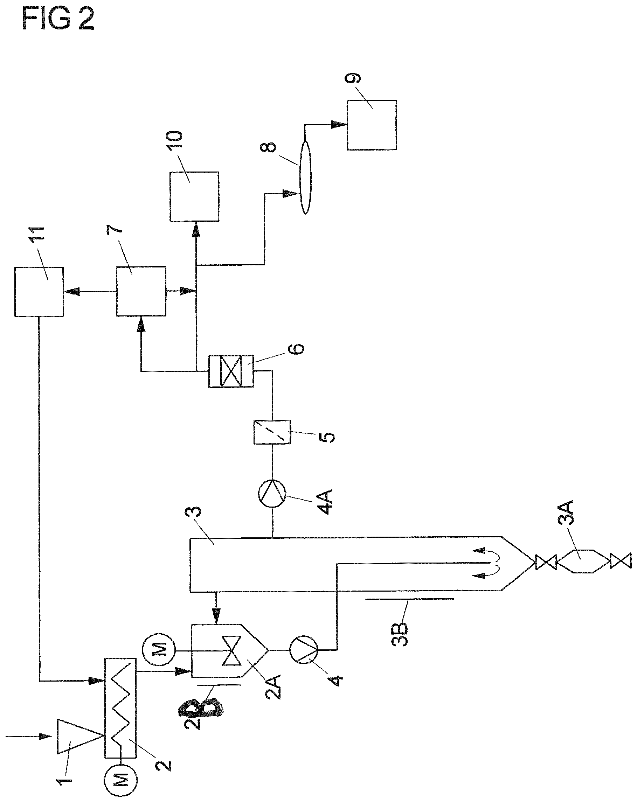

Figur 2 zeigt eine zweite Ausführungsform eines nicht erfindungsgemäßen Verfahrens, das sich von der ersten Ausführungsform der Figur 1 in Hinblick auf die Betriebsweise des Reaktors 3 unterscheidet.

Wie auch in Figur 1 wird das zerkleinerte Abfall-Polymer zunächst mit einem Reaktant oder Lösungsmittel in einer Zuführvorrichtung 2 gemischt. Das Gemisch wird anschließend jedoch zunächst einem Anmischbehälter 2A eingeleitet, in dem eine bessere Durchmischung von zerkleinerten Abfall-Polymer und Reaktant oder Lösungsmittel erreicht wird.

Diese Mischung verlässt den Anmischbehälter 2A und wird über eine geeignete Leitung in den unteren Bereich des Depolymerisationsreaktors 3 eingeleitet, der in diesem Ausführungsbeispiel kein Rührwerk aufweist. Die Mischung tritt entsprechend aus der Leitung an deren unterer Öffnung unmittelbar in die untere Reaktionszone des Reaktors 3 ein und strömt von unten nach oben durch den Depolymerisationsreaktor 3.

Der Abbau bzw. die Depolymerisation des Abfall-Polymers erfolgt auch hier bei erhöhter Temperatur. Die Erwärmung erfolgt dabei über eine innen- und außenliegende geeignete Heizung, beispielsweise 3B.

Die sich während der Depolymerisation im Reaktor abscheidenden Reststoffe werden am unteren Ende des Depolymerisationsreaktor 3 über eine Vorrichtung 3A zur Reststoffentleerung abgeführt. Dies hat den Vorteil, dass bereits im Reaktor ungewünschte grobe Feststoffe aus der Monomer-Lösung abgetrennt werden.

Die gebildete Monomerflüssigkeit wird am oberen Ende des Depolymerisationsreaktors 3 abgeführt und wie für Figur 1 beschrieben weiterverarbeitet.

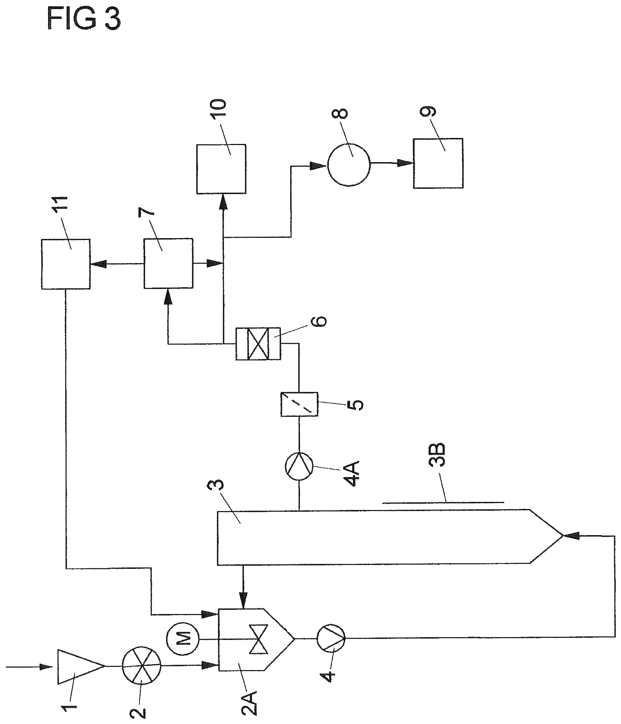

Die dritte nicht erfindungsgemäße Ausführungsform der Figur 3 unterscheidet sich von der zweiten Ausführungsform der Figur 2 lediglich in der Art der Zuführung der Mischung aus Abfall-Polymer und Reaktant oder Lösungsmittel in den Depolymerisationsreaktor 3.

Hier wird die Mischung aus Abfall-Polymer und Reaktant oder Lösungsmittel aus dem Anmischbehälter 2A direkt am unteren Ende des Reaktors 3 von außen zugeführt. Die Zuführleitung ist entsprechend vollständig außerhalb entlang der Reaktorlänge angeordnet. Auch hier weist der Depolymerisationsreaktor 3 kein Rührwerk auf und die Mischung durchströmt den Reaktor von unten nach oben.

Die gebildete Monomerflüssigkeit wird am oberen Ende des Reaktors 3 abgeführt und wie für Figur 1 beschrieben weiterverarbeitet.

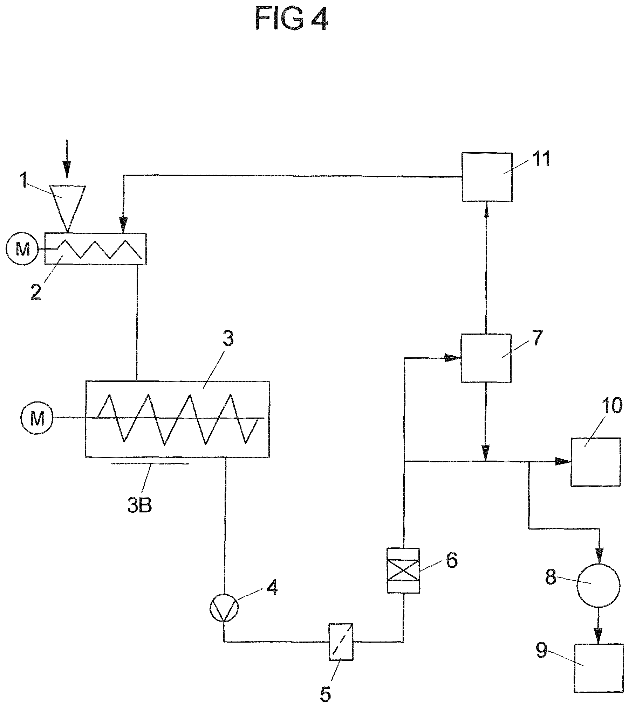

In Fig. 4 ist eine weitere nicht erfindungsgemäße Ausführungsform einer Vorrichtung und eines Verfahrens dargestellt, bei der der Reaktor 3 nicht vertikal - wie in den Ausführungsformen der Fig. 1 bis 3 dargestellt - sondern horizontal ausgebildet. Grundsätzlich kann somit auf die Beschreibungen der obigen Ausführungsformen Bezug genommen werden.

Anders als die vertikalen Ausführungsformen ist bei der horizontalen Ausführung des Reaktors 3 eine Schnecke als Misch- und / oder Fördereinrichtung vorgesehen. Die Schnecke kann dabei konzentrisch, wie in Fig. 4 dargestellt angeordnet sein, oder exzentrisch.

### Bezugszeichenliste

- 1: Aufgabevorrichtung für Abfall-Polymer
- 2: Zuführvorrichtung
- 2A: Anmischbehälter
- 2B: Heizung für Anmischbehälter
- 3: Depolymerisations-Lösereaktor
- 3A: Reststoffentleerung
- 3B: Heizung für Depolymerisations-Lösereaktor
- 4: Speisepumpe
- 4A: Austragpumpe
- 5: Einrichtung zur Abtrennung von unlöslichen Teilen
- 6: Entfärbungsreaktor
- 7: Konzentration- / Eindickungsvorrichtung
- 8: Verfestigungsvorrichtung
- 9: Verpackungsvorrichtung
- 10: Polymerisationsvorrichtung
- 11: Lösungsmittelaufbereitung / Reaktatenaufbereitung Schnecke

## Patentansprüche

1. Kontinuierliches Verfahren zur Depolymerisation und Lösung eines bezüglich der Partikelgröße und / oder Partikeldicke inhomogenen Polymergemisches unter Ausbildung einer im Wesentlichen aus einem Monomer bestehenden Monomerflüssigkeit, die mindestens einen Reaktant oder Lösungsmittel enthalten kann;
umfassend die Schritte
a) Einführen des inhomogenen Polymergemisches mit oder ohne Reaktant oder Lösungsmittel in mindestens einen vertikalen oder horizontalen Reaktor (3) mit mindestens einem Reaktoreinlass und mindestens einem Reaktorauslass und mit Einbauten zur Ausbildung von mindestens zwei Reaktionszonen innerhalb des Reaktors,,
b) kontinuierliche Depolymerisation und Lösung des inhomogenen Polymergemisches zu der Monomerflüssigkeit in dem mindestens einen vertikalen oder horizontalen Reaktor (3), wobei sich die Partikelgröße und / oder Partikeldicke des inhomogenen Polymergemisches entlang der Länge des Reaktors (3) von Reaktoreinlass zum Reaktorauslass durch Depolymerisation und Lösung kontinuierlich verringert,
wobei Partikel mit geringer Partikelgröße und / oder geringer Partikeldicke des inhomogenen Polymergemisches nach Eintritt in den Reaktor sofort depolymerisiert und gelöst werden und die gebildete Monomerflüssigkeit durch die Einbauten hin zum Reaktorauslass geführt und am Reaktorauslass aus dem Reaktor abgeführt wird, und
wobei Partikel großer Partikelgröße und / oder Partikeldicke des inhomogenen Polymergemisches nach Eintritt in den Reaktor durch die Einbauten zurückgehalten werden und solange im Reaktor verweilen bis diese Partikel des Polymergemisches vollständig depolymerisiert und gelöst sind.

2. Verfahren nach Anspruch 1, **dadurch gekennzeichnet, dass** das inhomogene Polymergemisch in mindestens einen vertikalen Reaktor (3) mit mindestens zwei Reaktionszonen mit jeweils mindestens einer Mischeinrichtung und / oder Fördereinrichtung eingeführt wird, wobei das inhomogene Polymergemisch den vertikalen Reaktor (3) von oben nach unten durchströmt.

3. Verfahren nach Anspruch 1, **dadurch gekennzeichnet, dass** das inhomogene Polymergemisch in mindestens einen vertikalen Reaktor (3) eingeführt wird, wobei das Polymer den vertikalen Reaktor (3) von unten nach oben durchströmt.

4. Verfahren nach Anspruch 1, **dadurch gekennzeichnet, dass** das inhomogene Polymergemisch in mindestens einen vertikalen Reaktor (3) eingeführt wird, wobei das Polymer den vertikalen Reaktor (3) mit jeweils mindestens einer Mischeinrichtung und / oder Fördereinrichtung von unten nach oben durchströmt.

5. Verfahren nach Anspruch 1, **dadurch gekennzeichnet, dass** das inhomogene Polymergemisch in mindestens einen horizontalen Reaktor (3), der eine Misch- und / oder Fördereinrichtung, insbesondere eine Förderschnecke aufweist, eingeführt wird, wobei das inhomogene Polymergemisch in den horizontalen Reaktor (3) mit oder entgegen der Strömungsrichtung arbeitenden Fördereinrichtung eingeleitet wird und die Monomerflüssigkeit am Reaktorende abgeführt wird.

6. Verfahren nach einem der vorhergehenden Ansprüche, **dadurch gekennzeichnet, dass** das inhomogene Polymergemisch Polymerpartikel mit einer Größe, insbesondere mittleren Größe in einem Bereich zwischen 30 mm und 1 mm, bevorzugt zwischen 15 mm und 3 mm aufweist oder Polymerpartikel mit einer Größe, insbesondere mittleren Partikelgröße zwischen 5 µm und 1 mm, insbesondere zwischen 10 µm und 0,5 mm aufweist.

7. Verfahren nach einem der vorhergehenden Ansprüche, **dadurch gekennzeichnet, dass** als Reaktant oder Lösungsmittel ein organischer Stoff, insbesondere ein organisches Lösungsmittel, ganz insbesondere ein Diol wie Ethylenglykol, oder eine wässrige Lösung, insbesondere eine saure wässrige Lösung verwendet wird.

8. Verfahren nach einem der vorhergehenden Ansprüche, **dadurch gekennzeichnet, dass** das inhomogene Polymergemisch ein Abfall-Polymergemisch, insbesondere aus Polyester und / oder Polyamiden, aus Industrie- und / oder Verbraucherabfällen ist.

9. Verfahren nach einem der vorhergehenden Ansprüche, **dadurch gekennzeichnet, dass** die Depolymerisation und / oder Lösung des inhomogenen Polymergemisches in dem Reaktor (3) bei Temperaturen zwischen 50 °C bis 350°C, insbesondere zwischen 180°C und 240°C, und Drücken zwischen 0,05 MPa und 1 MPa (0,5 bar und 10 bar) absolut, insbesondere zwischen 0,08 MPa und 0,6 MPa (0,8 bar und 6 bar) absolut durchgeführt wird.

10. Verfahren nach einem der vorhergehenden Ansprüche, **dadurch gekennzeichnet, dass** die Monomerflüssigkeit in mindestens einer Entfärbungsstufe, insbesondere in einem Entfärbungsreaktor (6), entfärbt wird.

11. Vorrichtung zur Durchführung eines kontinuierlichen Verfahrens nach einem der Ansprüche 1, 5-10, wobei der Reaktor (3) horizontal angeordnet ist und mindestens zwei Reaktionszonen aufweist,
**dadurch gekennzeichnet, dass**
die mindestens zwei Reaktionszonen durch eine Schnecke (12) gebildet werden, wobei die Schnecke (12) mit Löchern versehen ist, und wobei die Größe der Löcher in Richtung Einlassende des Reaktors (3) zunimmt.

12. Vorrichtung nach Anspruch 11, **dadurch gekennzeichnet, dass** die Vorrichtung eine Einrichtung (5) zum Abtrennen von unlöslichen Substanzen aufweist.

13. Vorrichtung nach mindestens einem der Ansprüche 11 oder 12, **gekennzeichnet durch** mindestens einen nachgeschalteten Entfärbungsreaktor (6) zum Entfärben der Monomerflüssigkeit.

14. Vorrichtung nach mindestens einem der Ansprüche 11 bis 13, **gekennzeichnet durch** mindestens eine Trennvorrichtung (7), insbesondere zum Eindicken und / oder Konzentrieren, zum zumindest teilweisen Entfernen des Reaktanten oder Lösungsmittel aus der Monomerflüssigkeit.

## Claims

1. A continuous process for the depolymerization and dissolution of a polymer mixture inhomogeneous with respect to the particle size and/or particle thickness by forming a monomer liquid substantially consisting of a monomer which may contain at least one reactant or solvent,
comprising the steps
a) introducing the inhomogeneous polymer mixture with or without at least one reactant or solvent into at least one vertical or horizontal reactor (3) with at least one reactor inlet and at least one reactor outlet and internal fittings for forming at least two reaction zones within the reactor,
b) continuous depolymerization and dissolution of the inhomogeneous polymer mixture to obtain the monomer liquid in the at least one vertical or horizontal reactor (3), wherein the particle size and/or particle thickness of the inhomogeneous polymer mixture is reduced continuously along the length of the reactor (3) from the reactor inlet to the reactor outlet by depolymerization,
wherein particles with small particle size and/or small particle thickness of the inhomogeneous polymer mixture are depolymerized and dissolved immediately after entry into the reactor and the monomer liquid formed is guided by the internal fittings towards the reactor outlet and at the reactor outlet discharged from the reactor, and
wherein particles of large particle size and/or particle thickness of the inhomogeneous polymer mixture after entry into the reactor will be retained by the internal fittings and remain in the reactor, until these particles of the polymer mixture are depolymerized and dissolved completely.

2. The process according to claim 1, **characterized in that** the inhomogeneous polymer mixture is introduced into at least one vertical reactor (3) with at least two reaction zones with at least one mixing device and/or conveying device each, wherein the inhomogeneous polymer mixture flows through the vertical reactor (3) from top to bottom.

3. The process according to claim 1, **characterized in that** the inhomogeneous polymer mixture is introduced into at least one vertical reactor (3), wherein the polymer flows through the vertical reactor (3) from bottom to top.

4. The process according to claim 1, **characterized in that** the inhomogeneous polymer mixture is introduced into at least one vertical reactor (3), wherein the polymer flows through the vertical reactor (3) with at least one mixing device and/or conveying device each from bottom to top.

5. The process according to claim 1, **characterized in that** the inhomogeneous polymer mixture is introduced into at least one horizontal reactor (3) which includes a mixing and/or conveying device, in particular a conveying screw, wherein the inhomogeneous polymer mixture is introduced into the horizontal reactor (3) by means of a conveying device operating with or against the flow direction and the monomer liquid is discharged at the reactor end.

6. The process according to any of the preceding claims, **characterized in that** the inhomogeneous polymer mixture includes polymer particles with a size, in particular mean size in a range between 30 mm and 1 mm, preferably between 15 mm and 3 mm, or polymer particles with a size, in particular mean particle size between 5 µm or 1 mm, in particular between 10 µm and 0.5 mm.

7. The process according to any of the preceding claims, **characterized in that** as reactant or solvent an organic substance, in particular an organic solvent, quite particularly a diol such as ethylene glycol, or an aqueous solution, in particular an acid aqueous solution is used.

8. The process according to any of the preceding claims, **characterized in that** the inhomogeneous polymer mixture is a waste polymer mixture, in particular of polyester and/or polyamides, from industrial and/or consumer wastes.

9. The process according to any of the preceding claims, **characterized in that** the depolymerization and/or dissolution of the inhomogeneous polymer mixture is carried out in the reactor (3) at temperatures between 50 °C and 350 °C, in particular between 180 °C and 240 °C, and pressures between 0.05 MPA and 1 MPa (0.5 bar and 10 bar) absolute, in particular between 0.08 MPA and 0.6 MPa (0.8 bar and 6 bar) absolute.

10. The process according to any of the preceding claims, **characterized in that** the monomer liquid is decolourized in at least one decolourizing stage, in particular in a decolourizing reactor (6).

11. An apparatus for carrying out a continuous process according to any of the claims 1, 5-10, wherein the reactor (3) is arranged either horizontally or vertically and includes at least two reaction zones,
**characterized in that**
the at least two reaction zone are formed by a screw (12), wherein the screw (12) is provided with holes, and wherein the size of the holes increases in direction of the inlet end of the reactor (3).

12. The apparatus according to claim 11, **characterized in that** the apparatus includes a device (5) for separating insoluble substances.

13. The apparatus according to claim 11 or 12, **characterized by** at least one downstream decolourizing reactor (6) for decolourizing the monomer liquid.

14. The apparatus according to at least one of claims 11 to 13, **characterized by** at least one separating device (7), in particular for thickening and/or concentrating, for at least partly removing the reactant or solvent from the monomer liquid.

## Revendications

1. Procédé continu de dépolymérisation et de dissolution d'un mélange polymère non homogène en termes de grandeur de particules et/ou d'épaisseur de particules en réalisant un liquide monomère constitué sensiblement d'un monomère, qui peut contenir au moins un réactif ou un solvant ;
comprenant les étapes
a) d'introduction du mélange polymère non homogène avec ou sans réactif ou solvant dans au moins un réacteur (3) vertical ou horizontal avec au moins une entrée de réacteur et au moins une sortie de réacteur et avec des installations servant à réaliser au moins deux zones de réaction à l'intérieur du réacteur,
b) de dépolymérisation et de dissolution en continu du mélange polymère non homogène en le liquide monomère dans l'au moins un réacteur (3) vertical ou horizontal, dans lequel la taille de particules et/ou l'épaisseur de particules du mélange polymère non homogène diminue en continu le long de la longueur du réacteur (3) depuis l'entrée de réacteur jusqu'à la sortie de réacteur du fait de la dépolymérisation et de la dissolution,
dans lequel des particules avec une grandeur de particules moindre et/ou une épaisseur de particules moindre du mélange polymère non homogène sont immédiatement dépolymérisées et dissoutes après l'entrée dans le réacteur et le liquide monomère obtenu est guidé à travers les installations en direction de la sortie de réacteur et est évacué du réacteur au niveau de la sortie de réacteur, et
dans lequel des particules de taille de particules importante et/ou d'épaisseur de particules importante du mélange polymère non homogène sont retenues par les installations après l'entrée dans le réacteur et restent dans le réacteur jusqu'à ce que ces particules du mélange polymère soient totalement dépolymérisées et dissoutes.

2. Procédé selon la revendication 1, **caractérisé en ce que** le mélange polymère non homogène est introduit dans au moins un réacteur (3) vertical avec au moins deux zones de réaction avec respectivement au moins un système de mélange et/ou un système de convoyage, dans lequel le mélange polymère non homogène traverse le réacteur (3) vertical depuis le haut vers le bas.

3. Procédé selon la revendication 1, **caractérisé en ce que** le mélange polymère non homogène est introduit dans au moins un réacteur (3) vertical, dans lequel le polymère traverse le réacteur (3) vertical depuis le bas vers le haut.

4. Procédé selon la revendication 1, **caractérisé en ce que** le mélange polymère non homogène est introduit dans au moins un réacteur (3) vertical, dans lequel le polymère traverse le réacteur (3) vertical avec respectivement au moins un système de mélange et/ou un système de convoyage depuis le bas vers le haut.

5. Procédé selon la revendication 1, **caractérisé en ce que** le mélange polymère non homogène est introduit dans au moins un réacteur (3) horizontal, qui présente un système de mélange et/ou de convoyage, en particulier une vis sans fin convoyeuse, dans lequel le mélange polymère non homogène est acheminé dans le réacteur (3) horizontal avec le système de convoyage fonctionnant dans le sens inverse au sens d'écoulement et le liquide monomère est évacué au niveau de l'extrémité de réacteur.

6. Procédé selon l'une quelconque des revendications précédentes, **caractérisé en ce que** le mélange polymère non homogène présente des particules polymères avec une taille, en particulier une taille moyenne dans une plage entre 30 mm et 1 mm, de manière préférée entre 15 mm et 3 mm, ou présente des particules polymères avec une taille, en particulier une taille de particules moyenne entre 5 µm et 1 mm, en particulier entre 10 µm et 0,5 mm.

7. Procédé selon l'une quelconque des revendications précédentes, **caractérisé en ce qu'**une matière organique, en particulier un solvant organique, tout particulièrement un diol tel que de l'éthylène glycol, ou une solution aqueuse, en particulier une solution aqueuse acide, est utilisée en tant que réactif ou solvant.

8. Procédé selon l'une quelconque des revendications précédentes, **caractérisé en ce que** le mélange polymère non homogène est un mélange polymère de déchet, en particulier composé de polyester et/ou de polyamides, issu de déchets industriels et/ou après consommation.

9. Procédé selon l'une quelconque des revendications précédentes, **caractérisé en ce que** la dépolymérisation et/ou la dissolution du mélange polymère non homogène est effectuée dans l'absolu dans le réacteur (3) à des températures entre 50 °C et 350 °C, en particulier entre 180 °C et 240 °C, et à des pressions entre 0,05 MPa et 1 MPa (0,5 bar et 10 bar), en particulier dans l'absolu entre 0,08 MPa et 0,6 MPa (0,8 bar et 6 bar).

10. Procédé selon l'une quelconque des revendications précédentes, **caractérisé en ce que** le liquide monomère est décoloré en au moins une étape de décoloration, en particulier dans un réacteur de décoloration (6).

11. Dispositif servant à effectuer un procédé continu selon l'une quelconque des revendications 1, 5 à 10, dans lequel le réacteur (3) est disposé de manière horizontale et présente au moins deux zones de réaction,
**caractérisé en ce que**
les au moins deux zones de réaction sont formées par une vis sans fin (12), dans lequel la vis sans fin (12) est pourvue de trous, et dans lequel la taille des trous augmente en direction de l'extrémité d'entrée du réacteur (3).

12. Dispositif selon la revendication 11, **caractérisé en ce que** le dispositif présente un système (5) servant à séparer des substances insolubles.

13. Dispositif selon au moins l'une quelconque des revendications 11 ou 12, **caractérisé par** au moins un réacteur de décoloration (6) installé en aval servant à décolorer le liquide monomère.

14. Dispositif selon au moins l'une quelconque des revendications 11 à 13, **caractérisé par** au moins un dispositif de séparation (7), en particulier d'épaississement et/ou de concentration, servant à éliminer au moins en partie le réactif ou le solvant du liquide monomère.
